(19) Europäisches Patentamt
European Patent Office
Office européen des brevets

(11) EP 4 610 335 A1

# (12) EUROPEAN PATENT APPLICATION
published in accordance with Art. 153(4) EPC

(43) Date of publication:
**03.09.2025 Bulletin 2025/36**

(21) Application number: **22836305.7**

(22) Date of filing: **24.10.2022**

(51) International Patent Classification (IPC):
***C10L 1/02*** *(2006.01)* ***C11C 3/04*** *(2006.01)*
***C12P 7/649*** *(2022.01)*

(52) Cooperative Patent Classification (CPC):
**C10L 1/026; C11C 3/003; C12N 9/20; C12P 7/6454; C12P 7/649; C12P 17/04; C12P 17/06; C12Y 301/01003;** Y02E 50/10

(86) International application number:
**PCT/ES2022/070687**

(87) International publication number:
**WO 2024/089298 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Selabtec Sciences, SLU**
**08225 Terrassa (ES)**

(72) Inventors:
- **HERRERO DÁVILA, Lorenzo**
  **08225 Terrassa (Barcelona) (ES)**
- **BAYARRI FERRER, Natividad**
  **08225 Terrassa (Barcelona) (ES)**
- **ESTÉVEZ COMPANY, Carles**
  **08225 Terrassa (Barcelona) (ES)**
- **DERONCELÉ THOMAS, Víctor Manuel**
  **08225 Terrassa (Barcelona) (ES)**

(74) Representative: **Ponti & Partners, S.L.P**
**Edifici PRISMA**
**Av. Diagonal núm. 611-613, Planta 2**
**08028 Barcelona (ES)**

## (54) METHOD FOR PREPARING A BIOFUEL COMPOSITION

(57) The present invention relates to a process for producing a FAGE-based biofuel composition, the process being characterized by an enzymatic transesterification of fatty acid alkyl esters with glycerol formal. The invention is also directed to the biofuel composition obtained by said process as well as to the uses thereof.

EP 4 610 335 A1

## Description

### TECHNICAL FIELD OF THE INVENTION

The present invention relates to the field biofuel production. More specifically, the present invention is related to a process for producing FAGE-based biofuel compositions, the process being characterized by an enzymatic transesterification of fatty acid alkyl esters with glycerol formal. The invention is also directed to the biofuel compositions obtained by said process as well as to the uses thereof.

### BACKGROUND OF THE INVENTION

Over the last thirty years, biodiesel has become a commercial reality, being currently integrated within the supply chains of diesel production worldwide. Biodiesel (generally fatty acid methyl ester (FAME)), is an optimum replacement for conventional diesel which use in diesel engines has the object of reduction Green House Gases emission globally.

Nonetheless, FAME production still has a persistent market failure due to the unavoidable generation of crude glycerin as coproduct. Crude glycerin is a impure composite of glycerol and other components that, without further purification has limited commercial applications. Despite the number of different strategies developed to find applications for crude glycerin, still there aren't widespread routes for an effective valorization of this impure co-product.

The process herein described, entails the conversion of fatty acid methyl esters (FAME) into fatty acid glycerol formal esters (FAGE) via enzymatic transesterification as means to a chemical incorporation of the molecule of glycerol (main component of crude glycerin) into a high value chemical product. The use of FAME/FAGE compositions as fuel, has been previously reported, as a component for production of diesel. The present patent improves the state of the art by incorporating said FAME /FAGE as a component in mixtures of renewable hydrocarbons and, therefore, is an effective solution to develop a new generation of more sustainable biofuels. Similarly, the present invention provides value to crude glycerin through an environmentally benign methodology.

Gycerol is incorporated into FAME to be converted into FAGE through a glycerol formal molecule (GF). GF is the main component of an acetal mixture yielded from the conversion of oils and fats into FAME in the presence of crude glycerol, methylal and acid catalysts. Other relevant component of the acetals mixture, is Glycerol formyl-methoxymethane (GFoMoM) a compound with prospective applications as a fuel and other chemical uses. An important novel aspect of this invention is the use of lipolytic enzymes as transesterification catalysts and preferably in its immobilized forms, which have advantages from the process efficiency and recovery point of view for this process. The use of enzymes in this process is a flexible, efficient and cleaner strategy that the chemical approach previously reported.

There have been previous attempts describing the production of FAME/FAGE compositions. In the next bibliographic review there are also described a number of transesterification reactions of different raw materials into FAME mediated by lipolytic/lipase enzymes. However, the prior art has never disclosed reactions for the production of FAGE based on an enzymatic reaction.

EP2730567 shows, for instance, a process for simultaneously producing alkyl esters of fatty acids fatty acids, glycerol formal and FAGE. The chemical reaction disclosed in EP2730567 is mediated by a Titanium catalyst, thus not being an enzymatically driven reaction.

EP2049623 also discloses a chemical process for producing FAGE from rapeseed oil but, again, it does not teach the use of lipolytic enzymes to this end.

WO2014102796 describes a process and a reactor for transesterification with immobilized enzymes in the production of fatty acid alkyl esters for use, *inter alia,* as biofuels. According to this document, the fatty acid alkyl esters are FAME or FAEE. The process described herein does not use glycerol formal as staring material.

There is also literature describing different hydrolytic enzymes. For instance, EP287634 describes a lipase with improved thermo stability in both soluble and immobilized form.

KR10-2225519 discloses a modified *Candida antartica* lipase B with improved hydrolytic, esterification and amination activity.

US2015/0031097**,** reports the use of catalysts selected from lipases, phospolipases and esterases for esterification and transesterification reactions.

However, none of these documents disclose a transesterification process involving the specific transformation of FAME to FAGE mediated by an enzyme.

The present invention is based on the surprising discovery that certain lipolytic enzymes are not only capable of transforming fatty acid alkyl esters into FAGE in the presence of glycerol formal but especially in that they are specific for such kind of transesterification reaction, leading to a process which, due to its high FAGE yields, short reaction times and uncomplicated work-up, is industrially scalable.

## BRIEF DESCRIPTION OF THE FIGURES

**Figure 1:** represents a typical chromatogram by gas chromatography, that quantifies esters by area unit and employing octanol as internal standard for quantification of acetals in a FAME/FAGE plus 3v/v acetal blend. In the composition of the final product obtained according to the process of the invention, The acetal blend incorporated in this composition typically contains GFoMoM: 75-90 wt% and glycerol formal 10-25 wt%.

**Figure 2:** rate of conversion of FAME into FAGE in the transesterification reaction mediated by *Candida antarctica* lipase.

**Figure 3:** shows the composition of the acetal mixture during conversion mediated *by Candida antarctica* lipase at different moments in time quantified by gas chromatography.

**Figure 4:** rate for FAME into a complete conversion to FAGE in the transesterification reaction mediated by *Candida antarctica.*

**Figure 5:** rate of conversion of FAME into FAGE in the transesterification reaction mediated by *Thermomyces lanuginosus* lipase.

## DESCRIPTION OF THE INVENTION

The following definitions are provided in order to facilitate the understanding and of clarifying the meaning of certain terms within the context of the present invention.

**"Biofuel":** A solid, gas or liquid fuel derived entirely from biomass that can be used as a replacement of fossil fuel and for the case of gases and liquids mainly in automotive applications.

**"Biofuel composition":** it refers to the product obtained by the process of the invention, formed by a blend of its components which comprises at least FAGE; fatty acid alkyl esters, preferably FAME; and acetals including GFoMoM. The composition is useful as biofuel or as a component for production of diesel and renewable diesel blends.

**"Fatty acid glycerol formal Ester"** or **"FAGE":** Esters of fatty acids resulting from the esterification of a certain fatty acid (generally C1-C28) with glycerol formal. In the context of the invention, it is produced by transesterification of fatty acid alkyl esters, preferably FAME, with glycerol formal.

**"Substrate":** is the element or substance containing the compounds or molecules upon which the enzyme exerts its activity.

**"Fatty acid alkyl Ester":** esters of fatty acids resulting, in the presence of a catalyst, from the esterification reaction of a free fatty acid (generally C1-C28) and an alcohol, or from the transesterification reaction of triglyceride also, producing glycerol as co-product.

**"Fatty acid methyl ester" or "FAME":** it is a fatty acid alkyl ester where the alkyl group is a methyl.

**"Source of fatty acid alkyl esters":** Raw material, generally of natural origin, where fatty acid alkyl esters can be derived from, such as animal fats and miscellaneous vegetable oils.

**"Glycerol formal"** or **"GF":** Acetal of glycerol resulting from the reaction of glycerol with methylal, formic acid or other acetalizing agent in the presence of a catalyst.

**"$C_1$-$C_{28}$ alkyl radical":** linear or branched, preferably linear, saturated aliphatic radicals containing from 1 to 28 carbon atoms.

**"$C_1$-$C_{28}$ alkenyl radical":** linear or branched, preferably linear, unsaturated aliphatic radicals containing from 1 to 28 carbon atoms and having from 1 to 7 double bonds.

**"Animal fats":** Lipid fractions extracted from the processing animal matter, which mayor components are triglycerides and free fatty acids. Animals fats are usually solid at room temperature.

**"Vegetable oils":** Lipid fractions extracted from the processing seeds or vegetable matter, which mayor components are triglycerides and free fatty acids. Vegetable oils are usually liquid at room temperature.

**"Food wastes":** Any food, and inedible parts of food, removed from a supply chain to be recovered, valorized or disposed. In the context, of the present invention food waste refers to the fatty or oleaginous fraction of food wastes.

**"Used cooking-oils":** UCOs are oils and fats that have been used for cooking or frying in the food processing industry, restaurants, fast foods and at consumer level, in households.

**"Non-food crops":** it is a crop grown to produce goods for manufacturing, rather than food for consumption. In the context of the invention, it refers to the oleaginous fraction from said crops.

**"Algae oils":** Lipid fractions extracted from the processing of algae and microalgae, which major components

are triglycerides and free fatty acids. Algae oils are usually liquid at room temperature.

**"Oleochemical residues":** lipidic substance or co-product generated in the processing of fats and vegetable oils, which remains unused when the main components have been taken for its specific use.

**"Lipolytic enzyme":** Lipolytic enzymes are a genetically varied group of industrially or non-industrial enzymes capable of catalyzing both the hydrolysis and the synthesis of lipids as well as esterification and transesterification reactions in lipidic substrates. In the context of the present invention the relevant lipolytic enzymes are those with capacity for transforming by transesterification reaction fatty acid alkyl ester, preferably FAME, into FAGE.

***"Thermomyces lanuginosus* lipase":** the natural or modified lipolytic enzyme sourced from the thermophilic fungus *Thermomyces lanuginosus.*

***"Candida antarctica* lipase B":** the natural or modified lipolytic enzyme sourced from the yeast *Candida antarctica.*

***"Proteus sp. K107* lipase":** the natural or modified lipolytic enzyme sourced from the bacteria *Proteus sp.*

**"Immobilized enzyme":** enzyme attached to an inert, insoluble material in the reaction medium. In this way, it provides to the enzyme an increased mechanical resistance or to changes in temperature or pH. Immobilized enzymes remain in place throughout the reaction and facilitates their recovery and re-usage.

**"Lypolitic enzyme loading":** Amount of enzyme immobilized in a certain support generally expressed as mg protein/amount of solid or as activity units (U) in the alternative.

**"Aqueous microenvironment":** The minimum amount of water necessary to guarantee the activity of the lipolytic enzyme, in a hydrophobic environment.

**"Mixture of acetals":** blend of acetals where the main component is glycerol formal and secondarily GFoMoM.

**"GFoMoM":** acetal derived from glycerol formal where the free hydroxyl group of glycerol formal has reacted with methylal. This product is generated in the transacetalisation reaction and is represented as follows:

Scheme 1

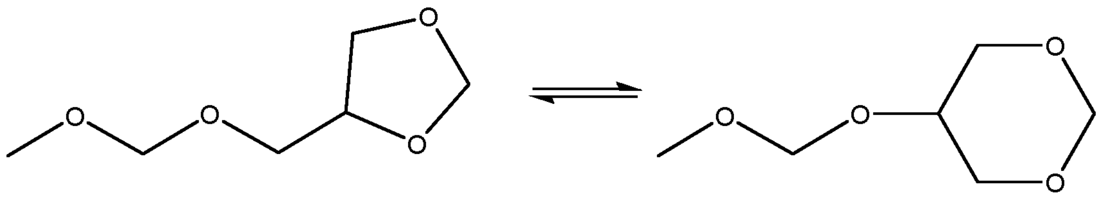

**"Recovered lipolytic enzyme":** immobilized enzyme separated, treated and regenerated from the crude of reaction which is reused again for a subsequent reaction.

**"Direct filtration":** processing step consisting in the separation of the immobilized enzyme by means of filtration with the aim of its subsequent treatment, regeneration and reuse.

**"Sequential washes":** in the context of enzyme recovery, the use of consecutive solvent washes to clean the lipolytic enzyme with the aim of being subsequently reused or disposed of.

**"Diesel":** types of fuels of defined specifications generally employed in internal combustion engines for automotive or of for heating purposes.

In a first aspect, the present invention is directed to a process for preparing a biofuel composition based on fatty acid glycerol formal esters (FAGE) of formula (I):

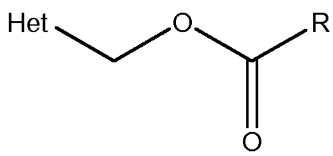

**(I)**

comprising the reaction of a substrate of fatty acid alkyl esters of formula (II):

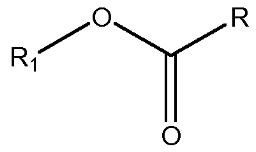

**(II)**

or a source of fatty acid alkyl esters of formula (II) with glycerol formal in the presence of at least one lipolytic enzyme. wherein

Het is a group selected from:

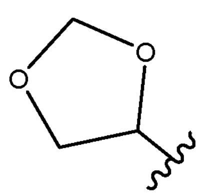 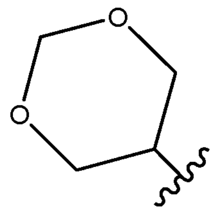

R is an $C_1$-$C_{28}$ alkyl radical or $C_1$-$C_{28}$ alkenyl radical; and
$R_1$ is an $C_1$-$C_6$ alkyl radical.

This process will be referred hereinafter either as the process according to the invention or simply the process of the invention.

In a particular and preferred embodiment, $R_1$ represents a methyl group resulting in that the fatty acid alkyl ester used as substrate for the reaction is a fatty acid methyl ester (FAME) of formula (IIa):

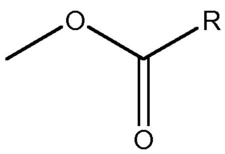

**(IIa)**

wherein R is as defined above.

The reaction between the compound of particularly preferred compound of formula (IIa) and glycerol formal according to the invention is schematically represented in scheme 1 below:

**Scheme 2**

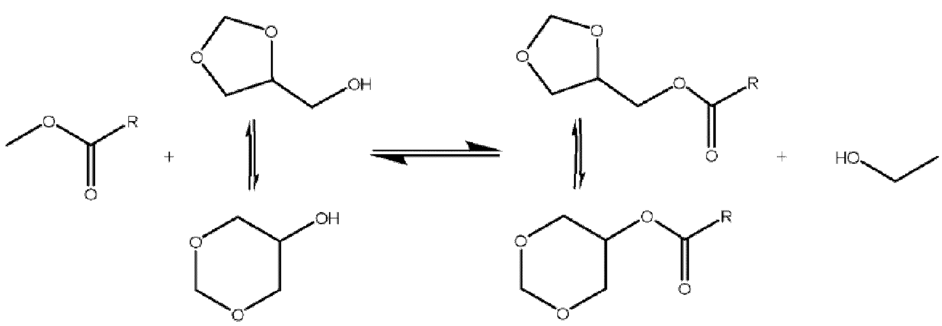

1 FAME 1 GF 1 FAGE 1 METHANOL

The substrate of the reaction can be a purified mixture of fatty acid alkyl esters of formula (II), preferably of formula

(IIa) or a source of fatty acid alkyl esters of formula (II) and/or of formula (IIa). Any lipidic source of fatty acid alkyl ester of formula (II) and/or of formula (IIa) is useful as a substrate for reaction in the process of the invention, although in a preferred embodiment the source of fatty acid alkyl esters of formula (II) and/or of formula (IIa) comprises at least one of animal fats, vegetable oils, food wastes, used cooking-oils, non-food crops, algae oils and oleochemical residues or mixtures thereof. The possibly of using any of these sources of fatty acid alkyl esters has the advantage of giving the process of the invention a great versatility and the possibility for reusing and recycling waste materials to give a product with an important added value.

One of the more characterizing aspects of the process of the invention is that it is an enzyme-mediated process. In particular, the transesterification reaction is driven by a lipolytic enzyme.

Although the inventors studied several non-commercial and commercially lipolytic enzymes well known for their transesterification capacity, they surprisingly found that the specific transesterification reaction between fatty acid alkyl esters of formula (II) and/or of formula (IIa) to yield a product enriched in fatty acid glycerol formal esters (FAGE) was extremely dependent on the type of lipolytic enzyme used. Most of the enzymes assayed were non-selective for this particular transesterification reaction or gave very poor FAGE transformation yields.

The inventors however have found that when the lipolytic enzyme was selected from *Thermomyces lanuginosa* lipase, *Candida antarctica* lipase B, *Proteus sp.* K107 Lipase or a mixture thereof, the reaction was not only very selective towards FAGE production but, in addition, yielded a reaction product with a high proportion of FAGE.

The lipolytic enzyme can be present in the reaction medium in free form, however, in a preferred embodiment the lipolytic enzyme is present in an immobilized form. The immobilized enzyme provides higher conversion rates and facilitates its manipulation after its use and its recovery for subsequent further uses. The inventors have found that the lipolytic enzyme after at least one recovery process preserves an enzymatic activity of more than 80% respect to the fresh enzyme.

For optimizing the reaction, the loading of the lipolytic enzyme or mixture of lipolytic enzymes in the reaction must be between 2-20wt.% by weight, preferably 2-10wt.% by weight, more preferably between 2-5wt.% by weight with respect to the fatty acid alkyl ester substrate.

In addition, in order to obtain the desired biofuel composition, the molar ratio between glycerol formal and the fatty acid alkyl ester substrate is especially relevant. In a particular and preferred embodiment of the invention, the molar ratio of glycerol formal to the fatty acid alkyl ester substrate is between 1-4 equivalents, preferably between 1-3 equivalents.

Glycerol formal can be obtained from crude glycerol via acetalysation process to yield a product with purity higher than 98%. The transformation of crude glycerol into glycerol formal with a purity higher than 98% can be carried out by any known process such as the one described, for instance, in DE19648960 using heterogeneous catalyst which allow to obtain a water free glycerol crude as the one described in WO 2005093015.

However, in a preferred embodiment of the invention, glycerol formal used in the process is not in pure form but generally present in a mixture of acetals. Said reactant mixture of acetals may contain of 1-99.9 wt.% of glycerol formal, more preferably between 30-75wt.% wt and even more preferably in 30-70wt.% by weight of the total mixture of acetals. The remaining amount of the mixture of acetals are generally glycerol formal derivatives such as GFoMoM (Glycerol formyl-methoxymethane).

Regarding the conditions of the process, the reaction is preferably carried out in an aqueous microenvironment with water in an amount of 0.01-1wt.% by weight with respect to the substrate of fatty alkyl acid esters or the source of fatty acid alkyl esters. Further although the process can be carried out at room temperature, it is preferred to carry out the reaction at a temperature between 40 and 70°C.

The process according to the invention can be carried in batch, continuous stirred-tank reactor, packed-bed column or expanded-bed reactor.

The process of the invention has the advantage that it allows optionally a further step of recovering the lipolytic enzyme for subsequent uses.

The recovery of the lipolytic enzyme comprises:

a) direct filtration, optionally followed by
b) sequential washes with organic solvents, such as acetone and heptane, and
c) drying under vacuum at a temperature of less than 40°C.

It is also another aspect of the invention the lipolytic enzyme recovered according to the process herein described. In a preferred embodiment the recovered enzyme is selected from *Thermomyces lanuginosus* lipase, *Candida antarctica* lipase B, *Proteus sp.* K107 Lipase, *Mucor miehe* lipase or a mixture thereof. The lipolytic enzyme after recovery process preserves an enzymatic activity of more than 80% respect to the fresh enzyme. This makes the recovered lipolytic enzyme perfectly suitable for further subsequent uses making the process of the invention highly sustainable.

Another aspect of the present invention is the biofuel composition obtainable by the process of the invention. This is going to be referred hereinafter as the biofuel composition of the invention or simply as the composition of the invention.

The biofuel composition of the invention is characterized by a comprising fatty acid glycerol formal esters (FAGE) of formula (I), fatty acid alkyl esters of formula (II), preferably of formula (IIa) and acetals.

Acetals present in the composition of the invention comprise glycerol formal and derivatives thereof, mainly GFoMoM.

The biofuel composition of the invention is defined by the ratio of their components. In particular embodiment, the composition has a ratio of fatty acid glycerol formal esters (FAGE)/ fatty acid alkyl esters/ acetals of 1-96.5/ 96.5-1/ 3-4; preferably of 46-50/ 50-46/ 3-4; and even more preferably 19-39/78-58/3-4.

Another aspect of the invention is related to the use of the biofuel composition of the invention.

The biofuel composition of the present invention has technical specifications compatible with commercial diesel for automotive in accordance with the diesel standard EN590 and with Hydrotreated Vegetable Oil standard (also known as renewable hydrocarbons or HVO) according to EN14590. Therefore, the composition can be used in adequate technical and quality requirements and it is suitable for use in diesel automotive engines.

The biofuel composition of the present invention meets the requirements in the current regulation to be considered a sustainable biofuel: it can be sourced from raw materials included in annex IX of the "UE Directive 2028/2001: European Directive on Renewable Energies" and it is compliant with the specifications and mixing conditions in place to meet the relevant quality standards for use as fuel for automotive.

The composition of the invention can be r used as a fuel component for the development and production of diesel and renewable diesel blends for automotive according to the aforementioned standards for conventional diesel, Hydrotreated Vegetable Oil (also known as renewable hydrocarbons or HVO), respectively or blends of those.

The invention will be described in more detail by means of the following examples. The description below discloses some embodiments and examples of the invention in such detail that a person skilled in the art is able to utilize the invention based on the disclosure. Not all the steps of the embodiments are disclosed in detail, as many of them will be obvious for a person skilled in the art.

## EXAMPLES

### EXAMPLE 1: Screening of lipolytic enzymes for the synthesis of glycerol formal fatty acid esters

Different commercial and non-commercia lipases (see Table 1) were screened for investigating the activity for the production of FAGE. Enzymatic transesterification reactions were performed using 800 µl of FAME (2.7mmol), 200 µl of GF (2.3mmol), 50 µl of water, and 3 wt.% of biocatalyst. The reactions were carried out at 40°C, using the multipoint reactor Carousel 12 plus reaction station (Radleys, Saffron Walden, UK). Reactions were conducted in 50 mL reaction tubes continuously mixed using a magnetic stirrer (200 rpm), and under vacuum in order to remove the methanol formed during the reaction, which shifted the reaction toward synthesis. To obtain the time course of the reaction, 50 µL of sample were taken at different time points and then analyzed via gas chromatography (GC-FID).

FAME and FAGE content were estimated by a correlation of area by using in this case, methyl heptadecanoate as internal standard following an adapted method from EN14103. The direct correlation between mass and response, with identical response factor for every methyl and every glycerol formyl esters is assessed. The analysis of FAME present in any oily sample is conducted by dissolving 1.00 g of the sample directly in 10mL of heptane and 10mL of the 2% (w/v) internal standard solution followed.

1 µL of the solution of 50mg/mL of FAME/FAGE or FAME mixture, were analyzed in a Shimadzu 2100, gas chromatograph fitted with a Zebron ZB-5MS GC capillary column 30 m x 0.25 mm x 0.25 µm. Injection temperature of 320°C, split ration 50:1 and FID temperature at 320°C, as auxiliar gas $N_2$ was used with a flow of 30mL/min, in addition to $H_2$ 40mL/min. Air 400mL/min. Total column flow 1mL/min. linear velocity 25.1cm/s. Carrier gas pressure (Helium) 80.9kPa.

The ramp employed was the following: T initial= 40° C held for 1 minute, subsequently rising at 10° C /min. to 150° C then held for 3 min., followed by two sequential ramps of 5° C/min. each, first to 200° C (held for 5mins.), second and finally to 320° C (held for 5 minutes), with a total run of 59 minutes.

Samples of 50 µl were diluted in 950 µl of the internal standard solution of methyl heptadecanoate prepared in heptane, and 1 µl was injected in a split ratio of 1:50. The conversion rate (%) was calculated with Eq. (1):

$$conversion\ rate\ (\%) = \frac{\frac{\sum \mathbf{fage}}{\mathbf{Amhd\ x\ Fr}}}{\frac{\sum \mathbf{fage}}{\mathbf{Amhd\ x\ Fr}} + \frac{\sum \mathbf{fame}}{\mathbf{Amhd}}}\ x\ 100 \qquad (1)$$

where, **Σfage** is the sum of all FAGE peak areas; **Amhd** is peak area for methyl heptadecanoate (internal standard); **Σfame** is the sum of all FAME peak areas, and **Fr** is the response factor.

As shown in Table 1 below, most of lipases rarely produced the FAGE compound, while Novozym 435 (*Candida antarctica*) showed the higher catalytic activity.

**Table 1. Lipase-catalyzed synthesis of FAGE[a]**

| Source | Regioselective | Conversion rate[b] (%) |
|---|---|---|
| *Pseudomonas fluorescens* | Non-selective | ND |
| *Thermomyces lanuginosus* | *sn-1,3* | 11.14 |
| *Mucor miehei* | *sn-1,3* | 2.28 |
| *Rhizopus oryzae* | *sn-1,3* | ND |
| *Candida rugosa* | Non-selective | ND |
| *Candida antarctica* | Non-selective | 40.85 |
| *Pseudomonas cepacia* | Non-selective | ND |
| *Rhizopus niveus* | *sn-1,3* | ND |
| *Rhizopus arrhizus* | *sn-1,3* | ND |
| *Penicillium camemberti* | Non-selective | ND |
| *Aspergillus oryzae* | *sn-1,3* | ND |
| *Lipoprotein-lipase from Pseudomonas sp.* | Non-selective | ND |
| *Lipoprotein-lipase from Burkholderia sp.* | Non-selective | ND |
| *Lipase from porcine páncreas* | *sn-1,3* | ND |
| *Lipase from Proteus sp.* K107 | Non-selective | 5.52 |

[a] Reaction conditions: FAME (2.7 mmol), GF (2.3 mmmol), water (50 ul); and lipases (0.03 g) were mixed using a magnetic stirrer (200 rpm), at 40 °C for 12 h.
[b] Determined by GC.

**EXAMPLE 2: Synthesis of FAME/FAGE mixture with the use of Eversa 2.0 (lipase from *Thermomyces Lanuginosus*).**

0.45g of FAME (1.5mmol), derived from used kitchen oil (Moisture <500ppm) together with 0.6g of glycerol formal (5.8mmol) were reacted in the presence of Eversa transform 2.0 (5wt% to FAME) for a period of 24h and 300rpms in orbital agitation at 37°C. The conversion of FAME to FAGE was verified by TLC and calculated by GC.

The conversion rate results of FAME to FAGE with Eversa 2.0 a lipase from *Thermomyces Lanuginosus* are shown in table 1 above.

**EXAMPLE 3: Synthesis of FAME/FAGE and GFoMoM in the same reaction with Novozyme 435 (*Candida antarctica* lipase B).**

2000 grams of FAME (6.76 mol), of water content below 500ppm, derived from a blend of animal fat and used kitchen oil were charged into a 4L reactor fitted with a vacuum distillation system. The system was closed and inerted with $N_2$, (0.8 bar) for up to 1 hour. The heating and the stirring were switched on (T=40-50°C, for the jacket reactor and 200rpm, respectively) and software the following addition curve for the acetal phase (containing 53% glycerol formal and 47% GFoMoM) was defined:

**Table 2: Addition curve for Acetal phase (reactant acetals mixture)**

| Time | Addition (mL/h) | Added GF vol (mL) | Added (9) | Rate (g/min) | Total GF added(g) | Total GFPhase added 8g) | Rate GFPhase (g/min.) | Rate GFPhase (mL/min.) | Rate GFPhase (mL/h) |
|---|---|---|---|---|---|---|---|---|---|
| **0** | Inst | 100 | Inst | inst | 120 | 226.2 | | | |
| **0-20** | 375 | 125 | 150 | 7.5 | 270 (9.5 mL/min, @ 5min and 12.0 mL/mn @ 20 min) | 508.9 | 14.13 | 12.29 | 737.47 |

(continued)

| Time | Addition (mL/h) | Added GF vol (mL) | Added (9) | Rate (g/min) | Total GF added(g) | Total GFPhase added 8g) | Rate GFPhase (g/min.) | Rate GFPhase (mL/min.) | Rate GFPhase (mL/h) |
|---|---|---|---|---|---|---|---|---|---|
| **20-40** | 275 | 91.67 | 110 | 5.5 | 380 | 716.2 | 10.36 | 9.01 | 540.81 |
| **40-60** | 250 | 83.33 | 100 | 5 | 480 | 904.6 | 9.42 | 8.19 | 491.65 |
| **60-80** | 225 | 75 | 90 | 4.5 | 570 | 1074.3 | 8.48 | 7.37 | 442.485 |
| **80-110** | 200 | 100 | 120 | 4 | 690 | 1300.4 | 7.53 | 5.56 | 393.32 |
| **110-140** | 175 | 87.5 | 105 | 3.5 | 795 | 1498.3 | 6.59 | 5.74 | 344.15 |
| **140-170** | 150 | 75 | 90 | 3 | 885 | 1667.9 | 5.65 | 4.92 | 294.99 |
| **230** | Inst | 100 | 73 | inst | 958 (9.21 mol) | 1805.5 | | | |

The reaction was conditioned for further 15 mins and 60g of Novozyme 435 was added. The vacuum was started at <30 mbar, as well as the addition of GF Phase as in Table 2 with the simultaneous collection of methanol via vacuum distillation system. Samples were collected by suction with $N_2$ 200rpm and 30mbar at t=0, 3=, 60, 120, 150, 180, 240, 300 mins. The composition of the crude was monitored in these times by GC for FAME, FAGE, GF, GFoMoM and FAGE. Figure 2 shows the kinetic rate of conversion of FAME into FAGE. In turn, Figure 3 shows the compositions of the acetal mixture during conversion at different moments in time.

Once the desired purity of FAGE was obtained in the mixture, the reaction is cooled to 25°C, with slow agitation (50rpm). The vacuum and the $N_2$ supply were stopped, as well as agitation.

The reactor was discharged, and enzyme was separated from the crude with a fritted filter no 3 under vacuum. The filtered enzyme was sequentially washed with up to three washes with acetone and the same number of times with heptane with the aim of being reused. The enzyme can be chosen to be reused directly.

The reaction crude was charged backed into a 2L spherical reactors (in two different batches) which was fitted with a vacuum distillation system. The system was inerted and fed with continuous flow of nitrogen to avoid as much as possible the oxidation of the fatty acids. Vacuum was then started and the GF Phase-enriched in GFoMoM (Total 112g, 75% by weight of GFoMoM, determined by GC) was distilled at 70-110°C at 3-10mbar.

Once distillation was finished temperature fell ca. 10° C. Acid Index was tested and corrected below 0.5 mg KOH/g for the distillation bottoms, via neutralization with a KOH 10% solution plus sequential water washes to remove excess alkalinity. The ester blend was then dried to <500ppm, under vaccum at 45°C for up to 24h, and then final purity of the FAME/FAGE composition was confirmed by GC as 70/30 wt% respectively.

**EXAMPLE 4: Synthesis of pure FAGE with Novozyme 435 (*Candida antarctica* lipase B).**

2500 grams of FAME (8.45 mol) of water content below 500ppm derived from a blend of vegetal oil and used kitchen oil, were charged into a 4L reactor fitted with a vacuum distillation system. The system was closed and inerted with $N_2$, (0.8 bar) for up to 1 hour. The heating and the stirring were switched on (T=50°C, for the jacket reactor (T=5°C) and 200rpm, respectively). The ester was conditioned for further 15 mins, then 75g of Novozyme 435 were added and then the addition curve (Table 3) for glycerol formal (Ultrapure Glycerol formal grade) was followed:

**Table 3: Addition curve for Glycerol formal**

| Time | Addition (mL/h) | Added GF vol (mL) | Added (9) | Rate (g/min) | Total GF added(g) |
|---|---|---|---|---|---|
| **0** | Inst | 125 | inst | inst | 125 |
| **0-20** | 468.75 | 156.25 | 187.5 | 9.38 | 312.5 |
| **20-40** | 343.75 | 114.58 | 137.5 | 6.88 | 450.0 |
| **40-60** | 312.5 | 104.17 | 125.00 | 6.25 | 575.0 |
| **60-80** | 281.25 | 9.75 | 112.50 | 5.63 | 687.5 |
| **80-110** | 250 | 125 | 150.00 | 5.00 | 837.5 |
| **110-140** | 218.75 | 109.38 | 131.25 | 4.38 | 968.8 |

(continued)

| Time | Addition (mL/h) | Added GF vol (mL) | Added (9) | Rate (g/min) | Total GF added(g) |
|---|---|---|---|---|---|
| **140-170** | 178.5 | 93.75 | 112.5 | 3.75 | 1081.3 |
| **170-200** | 156.25 | 78.13 | 93.75 | 3.13 | 1175.0 |
| **200--240** | 156.25 | 104.17 | 125.00 | 3.13 | 1300.0 |
| **240** | inst | 125.00 | 150.00 | Ints | 1450 .(13.92 mol) |

As the reaction starts, vacuum was set at <30mbar to continuously collect methanol via distillation system. The conversion was monitored by gas chromatography being samples collected at t=1, 4, 6, 8, 10, 14 and 23 hours. Beyond four hours addition of glycerol formal was stopped and conversion of FAME to FAGE was progressed and verified at 23 hours as 87%.

After that period, 100g more of glycerol formal (0.96 mol) were added over to the FAME/FAGE crude of reaction and conversion progressed for an additional period of 8 hours, until the conversion value of FAME to FAGE reached 96% (by GC). The total reaction time was 31 hours. The reaction was cooled down and the crude worked as in Example 3. Following reaction discharged, unreacted glycerol formal was quickly distilled of from the crude of reaction under vaccum and $N_2$ flow. Figure 4 shows the kinetic rate of conversion of FAME into FAGE.

Once distillation was finished purity of the FAME/FAGE distillation bottom was confirmed by GC. Acid index (mg KOH/g) (<0.5) and water content (<500ppm) were verified. If in need thereof acid index and water content were corrected as in Example 3.

Example 5: Synthesis of FAME/FAGE mixture and GFoMoM in the same reaction with *Thermomyces lanuginosus* lipase (Lipozyme TL IM).

1200 grams of FAME (4.05 mol) of water content below 500 ppm derived from used kitchen oil, were charged into a 4L reactor fitted with a vacuum distillation system. The system was closed and inerted with $N_2$, up to 1 hour. The heating and the stirring were switched on (T=50°C, for the jacket reactor and 200rpm, respectively) and the software defined the following addition curve for glycerol formal (<1000ppm of moisture and 99.9% purity) :

**Table 4: Addition curve for glycerol formal**

| Time | Addition (mL/h) | Added GF volume (mL) | Added (9) | Rate (g/min) | Total GF added (g) | Total GF(g) | Rate GF (g/min.) | Rate GF (mL/min.) | Rate GF (mL/h) |
|---|---|---|---|---|---|---|---|---|---|
| **0** | Inst | 5976 | inst | inst | 71.7 | 71.7 | | | |
| **0-20** | 224.1 | 747 | 89.64 | 4.48 | 161.4 | 161.4 | 4.48 | 39 | 233.84 |
| **20-40** | 164.34 | 54.78 | 65.74 | 3.29 | 227.1 | 227.1 | 3.29 | 2.86 | 171.90 |
| **40-60** | 149.4 | 49.8 | 59.76 | 2.99 | 286.8 | 286.8 | 2.99 | 2.60 | 155.9 |
| **60-80** | 134.46 | 44.82 | 53.78 | 2.69 | 340.6 | 340.6 | 2.69 | 2.34 | 140.31 |
| **80-110** | 119.52 | 59.76 | 71.71 | 2.39 | 412.3 | 412.3 | 2.39 | 2.08 | 124.72 |
| **110-140** | 104.58 | 52.29 | 62.75 | 2.09 | 475.1 | 475.1 | 2.09 | 1.82 | 109.13 |
| **140-170** | 89.64 | 44.82 | 53.78 | 1.79 | 528.9 | 528.9 | 1.79 | 1.56 | 93.54 |
| **230** | Inst | 59.76 | 73 | inst | 601.9 | 601.9 (5.78 mo) | | | |

The reaction was conditioned for further 15 mins and 234.9 g of Lypozyme TL IM of *Thermomyces lanuginosus* was added over a period of 30-60mins. The vacuum was started at <30mbar, as well as the addition of glycerol formal as in table 3, with the simultaneous collection of methanol via vacuum distillation system. Samples were collected by suction with $N_2$ 200rpm and 30mbar, at t=0, 3=, 60, 120, 150, 180, 240, 300 mins, similarly as to examples 2 and 3. The composition

of the crude was monitored by GC for FAME, FAGE and remaining GF. Figure 5 shows the kinetic rate of conversion of FAME into FAGE.

After 5 hours conversion did not seem to progress, the reaction was cooled to 25°C, with slow agitation (50rpm). The vacuum and the $N_2$ supply were stopped, as well as agitation.

**Example 6: Synthesis of FAME/FAGE with a blend of *Candida antarctica* lipase (Novozyme 435) and *Thermomyces lanuginosus* lipase (Lypozyme TL IM)**

The following proportions of Lypozyme TL IM and Novozyme 435 were added in a round bottom flask to made up 10wt% to 2.5g (8.44 mmol) of FAME (<500ppm moisture):

**Table 5**

| Trial | Catalyst |
|---|---|
| | |
| 1 | 0%Novozyme 435;10% Lypozyme TL IM |
| 2 | 0.1%Novozyme 435; 9.9% Lypozyme TL IM |
| 3 | 1%Novozyme 435; 9% Lypozyme TL IM |
| 4 | 5%Novozyme 435; 5% Lypozyme TL IM |
| 5 | 10%Novozyme 435; 0% Lypozyme TL IM |

The mixture starts a relax with agitation (200rpm) at 50°C with a continuous flow of $N_2$ for an initial time of 30mins. Glycerol formal (<1000ppm moisture), was sequentially added drop wise at a rate of 100μl after every 5 minutes for a total period of 45 minutes. After the addition time the reflux under nitrogen was kept for additional 30 minutes.

Apparent conversion of FAME to FAGE was qualitatively evaluated by TLC with the following results:

**Conversion 1< Conversion 2< Conversion 3< Conversion 5< Conversion 4**

These results are indicative that *Candida antarctica* lipase provides higher conversion rates than *Thermomyces lanuginosus* lipase, when used alone or with small proportion of *Thermomyces lanuginosus* lipase but at the same time reveals a synergistic effect in conversion when used in combination at a balanced proportion.

## Claims

1. A process for preparing a biofuel composition based on fatty acid glycerol formal esters (FAGE) of formula (I):

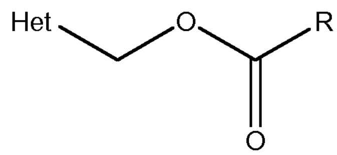

**(I)**

comprising the reaction of a substrate of fatty acid alkyl esters of formula (II):

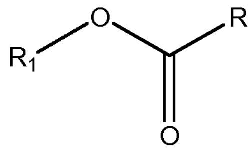

**(II)**

or a source of fatty acid alkyl esters of formula (II) with glycerol formal in the presence of at least one lipolytic enzyme,
wherein
Het is a group selected from:

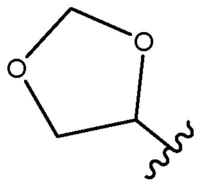

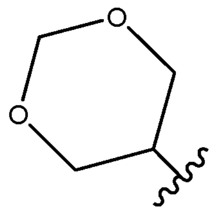

R is an $C_1$-$C_{28}$ alkyl radical or $C_1$-$C_{28}$ alkenyl radical; and
$R_1$ is an $C_1$-$C_6$ alkyl radical.

**2.** A process according to claim 1 wherein the fatty acid alkyl ester is a fatty acid methyl ester (FAME) of formula (IIa):

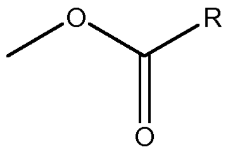

**(IIa)**

wherein R is as defined in claim 1.

**3.** A process according to claim 1 wherein the source of fatty acid alkyl esters of formula (II) comprises at least one of animal fats, vegetable oils, food wastes, used cooking-oils, non-food crops, algae oils and oleochemical residues.

**4.** A process according to any of the previous claims wherein the lipolytic enzyme is selected from *Thermomyces sp.* lipase; *Candida sp.* lipase, *Proteus sp.* lipase, *Mucor sp.* lipase or a mixture thereof.

**5.** A process according to any of the previous claims wherein the lipolytic enzyme is selected from *Thermomyces lanuginosus lipase; Candida antarctica lipase B, Proteus sp. K107 Lipase, Mucor miehe* lipase or a mixture thereof.

**6.** A process according to any of the previous claims wherein the lipolytic enzyme is in an immobilized form.

**7.** A process according to any of the previous claims wherein the lipolytic enzyme loading in the reaction is between 2-20wt.% by weight, preferably 2-10wt.% by weight, more preferably between 2-5wt.% by weight with respect to the fatty acid alkyl ester substrate.

**8.** A process according to any of the previous claims wherein the molar ratio of glycerol formal to the fatty acid alkyl ester substrate is between 1-4 equivalents, preferably between 1-3 equivalents.

**9.** A process according to any of the previous claims wherein the reaction is carried out in an aqueous microenvironment with water in an amount of 0.01-1wt.% by weight with respect to the substrate of fatty alkyl acid esters.

**10.** A process according to any of the previous claims wherein the reaction is carried out at a temperature between 40 and 70°C.

**11.** A process according to any of the previous claims wherein glycerol formal is present in a mixture of acetals.

**12.** A process according to claim 10, wherein the mixture of acetals may contain of 1-99.9 wt.% of glycerol formal, more preferably between 30-75wt.% wt and even more preferably in 30-70wt.% by weight of the total mixture of acetals.

**13.** A process according to any of the previous claims wherein the process is carried out in batch, continuous stirred-tank reactor, packed-bed column or expanded-bed reactor.

**14.** A process according to any of the previous claims comprising a further step of recovering the lipolytic enzyme for subsequent uses.

**15.** A process according to claim 14 wherein the recovery of the lipolytic enzyme comprises:

a) direct filtration, optionally followed by
b) sequential washes with organic solvents
c) drying under vacuum at a temperature of less than 40°C

**16.** A biofuel composition obtainable by the process of any of claims 1-15 **characterised in that** it comprises fatty acid glycerol formal esters (FAGE) of formula (I), fatty acid alkyl esters of formula (II) as defined in claim 1 and acetals.

**17.** A biofuel composition according to claims 16 where acetals comprise glycerol formal and GFoMoM.

**18.** A biofuel composition according to claim 17 wherein the ratio of fatty acid glycerol formal esters (FAGE)/ fatty acid alkyl esters/ acetals is 1-96.5/ 96.5-1/ 3-4; preferably 46-50/ 50-46/ 3-4; and even more preferably 19-39/78-58/3-4.

**19.** Use of a biofuel composition according to any of claims 15 to 17 as a biofuel.

**20.** Use of a biofuel composition according to any of claims 15 to 17 as a component for production of diesel and renewable diesel blends.

**21.** A lipolytic enzyme recovered according to the process of any of claims 14 or 15.

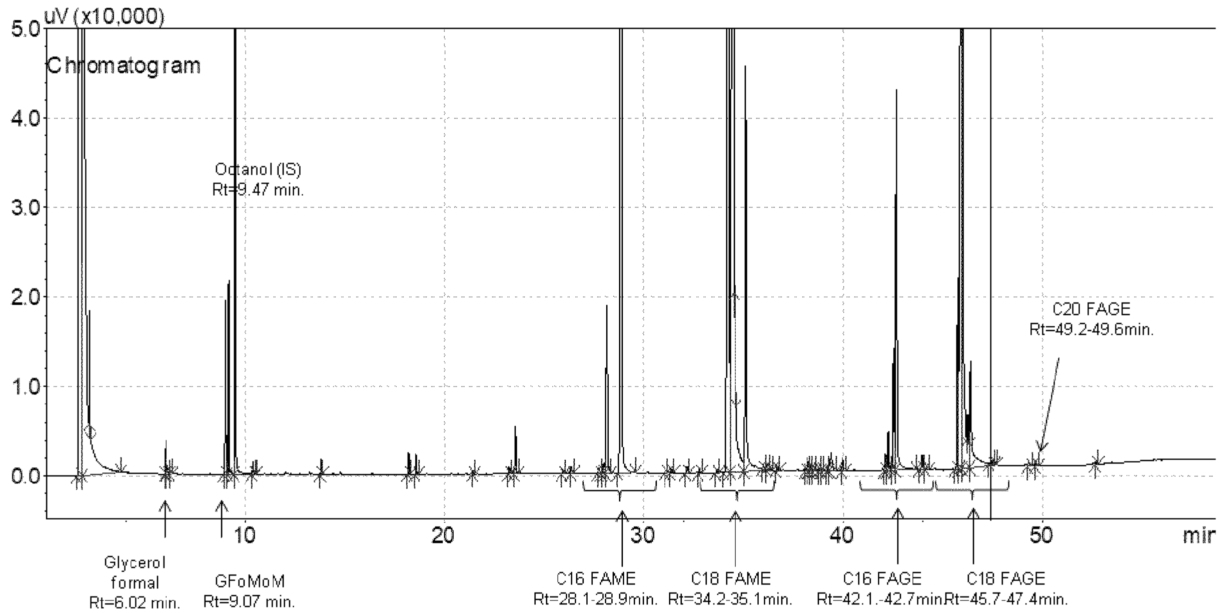

uV (x10,000)
Chromatogram
5.0
4.0
3.0
2.0
1.0
0.0
10
20
30
40
50
min
Octanol (IS)
Rt=9.47 min.
C20 FAGE
Rt=49.2-49.6min.
Glycerol formal
Rt=6.02 min.
GFoMoM
Rt=9.07 min.
C16 FAME
Rt=28.1-28.9min.
C18 FAME
Rt=34.2-35.1min.
C16 FAGE
Rt=42.1.-42.7min
C18 FAGE
Rt=45.7-47.4min.


Fig. 1

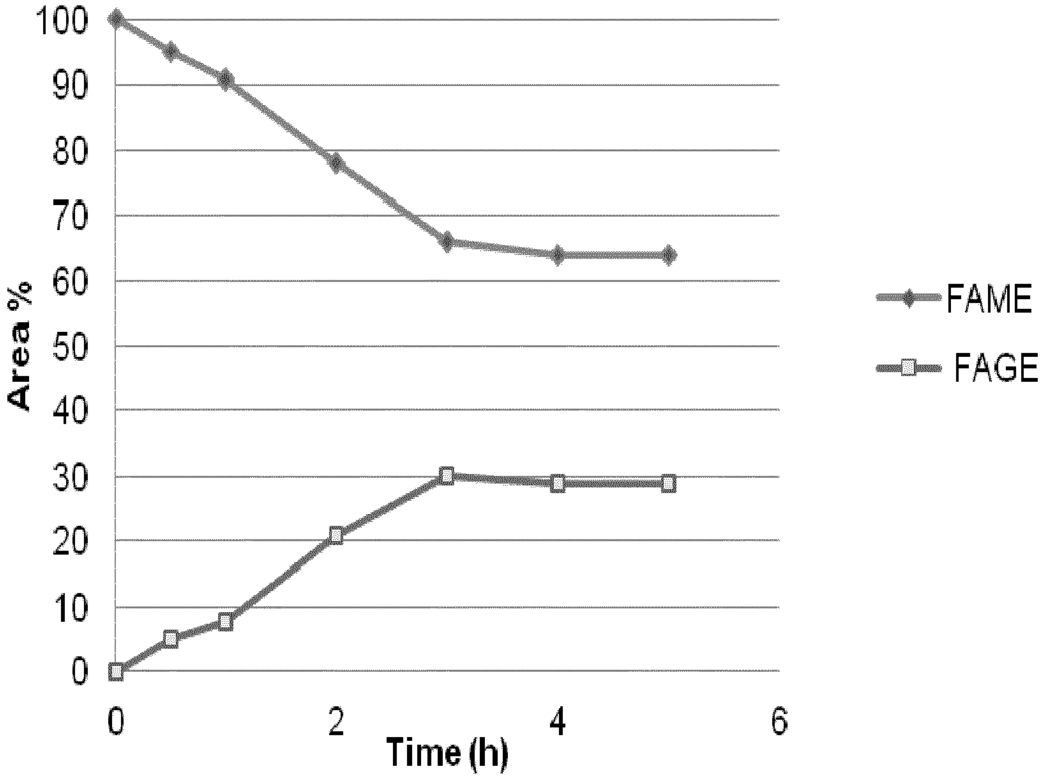


Fig. 2

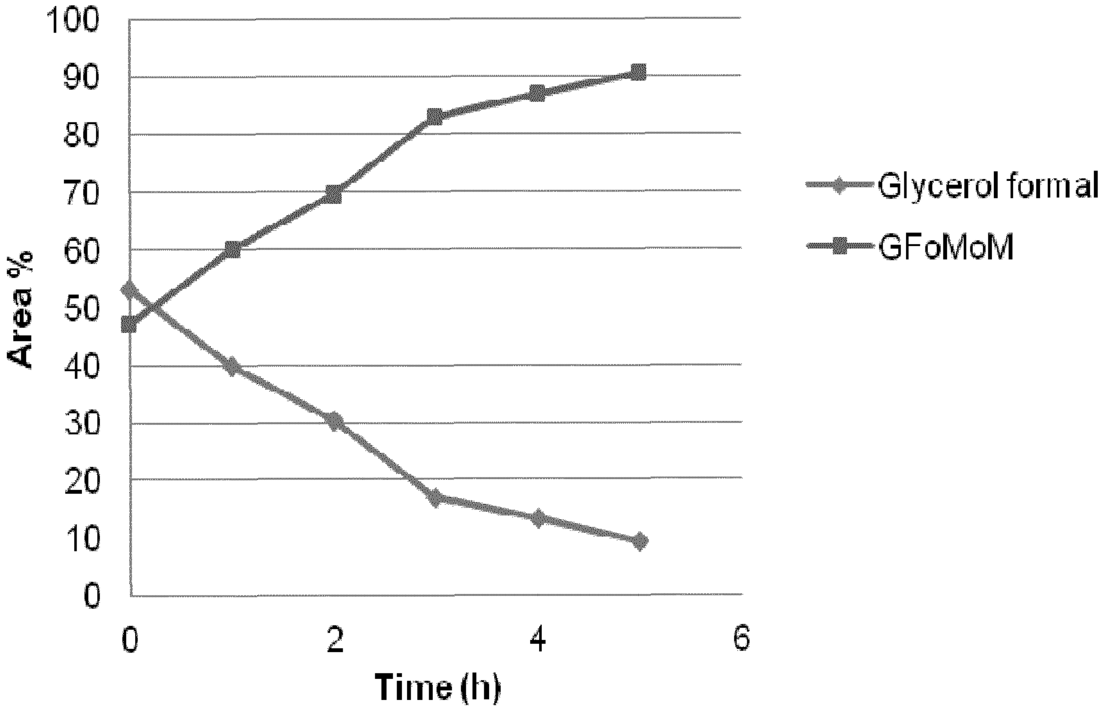
100
90
80
70
60
50
40
30
20
10
0
Area %
0
2
4
6
Time (h)
Glycerol formal
GFoMoM

Fig. 3

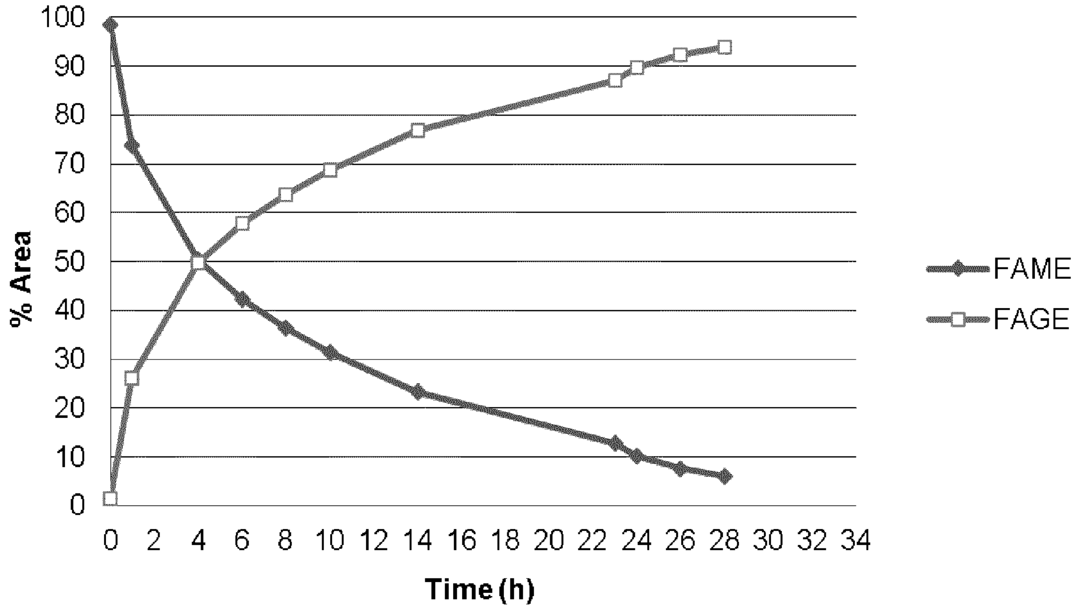
100
90
80
70
60
50
40
30
20
10
0
% Area
0 2 4 6 8 10 12 14 16 18 20 22 24 26 28 30 32 34
Time (h)
FAME
FAGE

# Fig. 4

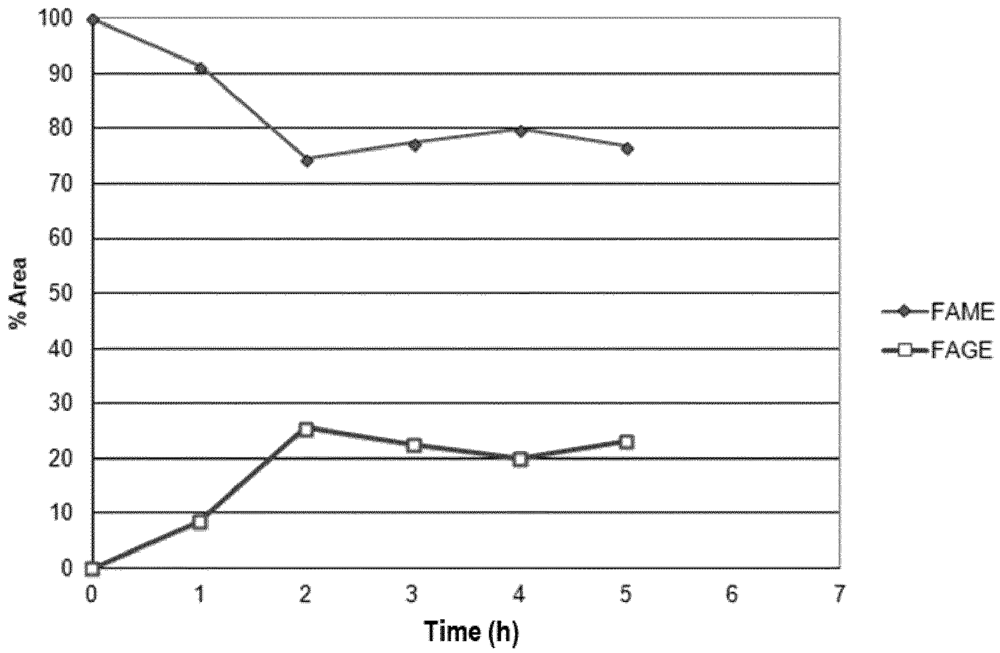


# Fig. 5

## INTERNATIONAL SEARCH REPORT

International application No
PCT/ES2022/070687

**A. CLASSIFICATION OF SUBJECT MATTER**
INV. C10L1/02 C11C3/04 C12P7/649
ADD.

According to International Patent Classification (IPC) or to both national classification and IPC

**B. FIELDS SEARCHED**

Minimum documentation searched (classification system followed by classification symbols)
C10L C11C C12P

Documentation searched other than minimum documentation to the extent that such documents are included in the fields searched

Electronic data base consulted during the international search (name of data base and, where practicable, search terms used)

EPO-Internal, WPI Data

**C. DOCUMENTS CONSIDERED TO BE RELEVANT**

| Category* | Citation of document, with indication, where appropriate, of the relevant passages | Relevant to claim No. |
|---|---|---|
| X | LAPUERTA MAGÍN ET AL: "Properties of fatty acid glycerol formal ester (FAGE) for use as a component in blends for diesel engines", BIOMASS AND BIOENERGY, vol. 76, 1 May 2015 (2015-05-01), pages 130-140, XP093039261, AMSTERDAM, NL ISSN: 0961-9534, DOI: 10.1016/j.biombioe.2015.03.008 Retrieved from the Internet: URL:https://www.sciencedirect.com/science/article/pii/S0961953415000884/pdfft?md5=7678a803c82b428a2360832b77184e7a&pid=1-s2.0-S0961953415000884-main.pdf> page 131 - page 133; tables 1,2 ----- -/-- | 1-21 |

[X] Further documents are listed in the continuation of Box C.

[X] See patent family annex.

* Special categories of cited documents :

"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance;; the claimed invention cannot be considered novel or cannot be considered to involve an inventive step when the document is taken alone
"Y" document of particular relevance;; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 April 2023 | 24/04/2023 |

| Name and mailing address of the ISA/ | Authorized officer |
|---|---|
| European Patent Office, P.B. 5818 Patentlaan 2 NL - 2280 HV Rijswijk Tel. (+31-70) 340-2040, Fax: (+31-70) 340-3016 | Deurinck, Patricia |

Form PCT/ISA/210 (second sheet) (April 2005)

## INTERNATIONAL SEARCH REPORT

International application No
PCT/ES2022/070687

C(Continuation). DOCUMENTS CONSIDERED TO BE RELEVANT

| Category* | Citation of document, with indication, where appropriate, of the relevant passages | Relevant to claim No. |
|---|---|---|
| X | LAPUERTA MAGÍN ET AL: "Improvement of cold flow properties of a new biofuel derived from glycerol", FUEL, vol. 242, 1 April 2019 (2019-04-01), pages 794-803, XP093039262, GB ISSN: 0016-2361, DOI: 10.1016/j.fuel.2019.01.066 Retrieved from the Internet: URL:https://www.sciencedirect.com/science/article/pii/S0016236119300687/pdfft?md5=423e1e1a0ab80154f37677eae0b8cd1e&pid=1-s2.0-S0016236119300687-main.pdf> paragraph [0002] - paragraph [0004] ----- | 16-20 |
| X | RODRÍGUEZ-FERNÁNDEZ JOSÉ ET AL: "Selection of Blends of Diesel Fuel and Advanced Biofuels Based on Their Physical and Thermochemical Properties", ENERGIES, vol. 12, no. 11, 28 May 2019 (2019-05-28), page 2034, XP093039272, DOI: 10.3390/en12112034 Retrieved from the Internet: URL:https://www.sciencedirect.com/science/article/pii/S0016236119300687/pdfft?md5=423e1e1a0ab80154f37677eae0b8cd1e&pid=1-s2.0-S0016236119300687-main.pdf> paragraph [0003] ----- | 16-20 |
| X | LAPUERTA MAGÍN ET AL: "Optimization of a diesel engine calibration for operating with a residual glycerol-derived biofuel", INTERNATIONAL JOURNAL OF ENGINE RESEARCH, vol. 22, no. 4, 1 April 2021 (2021-04-01), pages 1273-1284, XP093039150, GB ISSN: 1468-0874, DOI: 10.1177/1468087419891535 Retrieved from the Internet: URL:http://journals.sagepub.com/doi/full-xml/10.1177/1468087419891535> page 1275 - page 1276 ----- | 16-20 |
| X | WO 2008/006860 A2 (INST UNI DE CIENCIA I TECNOLOG [ES] ET AL.) 17 January 2008 (2008-01-17) example 3 ----- | 16-20 |
| X | EP 2 757 140 A1 (INST UNIV DE CIÈNCIA I TECNOLOGIA S A [ES]) 23 July 2014 (2014-07-23) claims 2,7 ----- | 16-20 |
| | -/-- | |

Form PCT/ISA/210 (continuation of second sheet) (April 2005)

## INTERNATIONAL SEARCH REPORT

International application No
PCT/ES2022/070687

C(Continuation). DOCUMENTS CONSIDERED TO BE RELEVANT

| Category* | Citation of document, with indication, where appropriate, of the relevant passages | Relevant to claim No. |
|---|---|---|
| X | LOZANO PEDRO ET AL: "Clean Enzymatic Preparation of Oxygenated Biofuels from Vegetable and Waste Cooking Oils by Using Spongelike Ionic Liquids Technology", ACS SUSTAINABLE CHEMISTRY & ENGINEERING, vol. 4, no. 11, 24 August 2016 (2016-08-24), pages 6125-6132, XP093039282, US ISSN: 2168-0485, DOI: 10.1021/acssuschemeng.6b01570 Retrieved from the Internet: URL:https://www.sciencedirect.com/science/article/pii/S0016236119300687/pdfft?md5=423e1e1a0ab80154f37677eae0b8cd1e&pid=1-s2.0-S0016236119300687-main.pdf> page 6127 ----- | 21 |
| X | WO 2010/049491 A1 (NOVOZYMES AS [DK]; BRASK JESPER [DK] ET AL.) 6 May 2010 (2010-05-06) claims 1,2 ----- | 21 |

Form PCT/ISA/210 (continuation of second sheet) (April 2005)

## INTERNATIONAL SEARCH REPORT

Information on patent family members

International application No
PCT/ES2022/070687

| Patent document cited in search report | | Publication date | Patent family member(s) | | | Publication date |
|---|---|---|---|---|---|---|
| WO 2008006860 | A2 | 17-01-2008 | AT | 510902 | T | 15-06-2011 |
| | | | AU | 2007274260 | A1 | 17-01-2008 |
| | | | BR | PI0713161 | A2 | 03-04-2012 |
| | | | CA | 2657431 | A1 | 17-01-2008 |
| | | | CN | 101541929 | A | 23-09-2009 |
| | | | CO | 6180468 | A2 | 19-07-2010 |
| | | | CR | 10586 | A | 29-04-2009 |
| | | | CY | 1111764 | T1 | 07-10-2015 |
| | | | DK | 2049623 | T3 | 05-09-2011 |
| | | | EP | 2049623 | A2 | 22-04-2009 |
| | | | ES | 2365226 | T3 | 26-09-2011 |
| | | | HK | 1129416 | A1 | 27-11-2009 |
| | | | IL | 196464 | A | 30-04-2015 |
| | | | JP | 5342441 | B2 | 13-11-2013 |
| | | | JP | 2010506960 | A | 04-03-2010 |
| | | | KR | 20090045243 | A | 07-05-2009 |
| | | | MY | 145007 | A | 15-12-2011 |
| | | | NZ | 574432 | A | 30-09-2010 |
| | | | PL | 2049623 | T3 | 30-11-2011 |
| | | | PT | 2049623 | E | 11-08-2011 |
| | | | SI | 2049623 | T1 | 30-09-2011 |
| | | | US | 2010005708 | A1 | 14-01-2010 |
| | | | WO | 2008006860 | A2 | 17-01-2008 |
| EP 2757140 | A1 | 23-07-2014 | AR | 092174 | A1 | 25-03-2015 |
| | | | AU | 2014206867 | A1 | 06-08-2015 |
| | | | BR | 112015017043 | A2 | 11-07-2017 |
| | | | CA | 2895912 | A1 | 24-07-2014 |
| | | | CN | 104937081 | A | 23-09-2015 |
| | | | CO | 7461147 | A2 | 30-11-2015 |
| | | | CR | 20150389 | A | 21-08-2015 |
| | | | DK | 2946001 | T3 | 22-11-2021 |
| | | | EC | SP15034307 | A | 30-11-2015 |
| | | | EP | 2757140 | A1 | 23-07-2014 |
| | | | EP | 2946001 | A1 | 25-11-2015 |
| | | | ES | 2895326 | T3 | 18-02-2022 |
| | | | HK | 1214620 | A1 | 29-07-2016 |
| | | | HR | P20211772 | T1 | 04-03-2022 |
| | | | HU | E056381 | T2 | 28-02-2022 |
| | | | IL | 239608 | A | 31-07-2017 |
| | | | JP | 6381548 | B2 | 29-08-2018 |
| | | | JP | 2016503830 | A | 08-02-2016 |
| | | | LT | 2946001 | T | 27-12-2021 |
| | | | MY | 169944 | A | 18-06-2019 |
| | | | PL | 2946001 | T3 | 14-02-2022 |
| | | | PT | 2946001 | T | 08-11-2021 |
| | | | RS | 62549 | B1 | 31-12-2021 |
| | | | SI | 2946001 | T1 | 31-03-2022 |
| | | | US | 2015353855 | A1 | 10-12-2015 |
| | | | WO | 2014111490 | A1 | 24-07-2014 |
| WO 2010049491 | A1 | 06-05-2010 | AR | 074019 | A1 | 15-12-2010 |
| | | | BR | PI0920048 | A2 | 25-08-2015 |
| | | | CN | 102272317 | A | 07-12-2011 |
| | | | EP | 2350301 | A1 | 03-08-2011 |
| | | | RU | 2011121868 | A | 10-12-2012 |
| | | | US | 2011219675 | A1 | 15-09-2011 |
| | | | WO | 2010049491 | A1 | 06-05-2010 |

Form PCT/ISA/210 (patent family annex) (April 2005)

## INTERNATIONAL SEARCH REPORT

Information on patent family members

International application No
PCT/ES2022/070687

| Patent document cited in search report | Publication date | Patent family member(s) | Publication date |
|---|---|---|---|

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- EP 2730567 A
- EP 2049623 A
- WO 2014102796 A
- EP 287634 A
- KR 102225519
- US 20150031097 A
- DE 19648960
- WO 2005093015 A

### Non-patent literature cited in the description

- *UE Directive 2028/2001: European Directive on Renewable Energies*